# NEUE EUROPÄISCHE PATENTSCHRIFT

(11) **EP 0 854 129 B2**
(45) Veröffentlichungstag und Bekanntmachung des Hinweises auf die Entscheidung über den Einspruch: **28.11.2007**
(45) Hinweis auf die Patenterteilung: 16.06.1999
(21) Anmeldenummer: 96116469.6
(22) Anmeldetag: 17.01.1997
(51) Int. Cl.: C07C 57/04, C07C 51/44, B01D 3/32

(54) **Verfahren der kontinuierlichen destillativen Auftrennung von flüssigen Gemischen, die als Hauptbestandteil (Meth)acrylsäure enthalten**
Process for the continuous separation by distillation of liquid mixtures containing (meth)acrylic acid as the main cut
Procédé de séparation par distillation continue de mélanges liquides contenant l'acide (méth)acrylique comme la fraction principale

(43) Veröffentlichungstag der Anmeldung: 22.07.1998
(73) Patentinhaber: BASF Aktiengesellschaft, 67056 Ludwigshafen (DE)
(72) Erfinder: Kroker, Ruprecht, Dr., 67240 Bobenheim-Roxheim (DE); Wiedemann, Manfred, 67071 Ludwigshafen (DE)

(56) Entgegenhaltungen:
- EP-A- 0 620 206
- EP-A- 1 084 740
- DE-A- 1 519 595
- DE-A- 2 104 890
- DE-B- 1 814 774
- FR-A- 1 421 185
- US-A- 4 261 798
- W.A. Chantry et al, Chem. Eng. Progress, 1958, 54(10), 64-67
- M.J. Fried, Farbe + Lack, 100, 8/1994, S. 604-609
- CD Römpp Chemie Lexikon, Version 1.0, 1995
- Plant/Operations Progress, Bd. 7, Nr. 3, 1988, 183-189
- Journal of Polymer Science, Bd. 23, 1985, 1505-1515
- European Polymer Journal, Bd. 17, 1981, 1197-1203
- Kirk-Othmer, Encyclopedia of Chemical Technology, 3. Aufl., 1978, Bd. 1, 349
- Beilsteins Handbuch der Org. Chemie, 4. Aufl., 3. Ergänzungswerk, 1961, Bd. 3, 1. Teil, EIII 3, 525
- Ullmann's Encyclopedia of Industrial Chemistry, 6. Aufl., 1999 Electronic Release, Kapitel: Acrylic Acid and Derivatives
- Ullmann's Encyclopedia of Industrial Chemistry, 5. Aufl., 1985, Bd. 1, 166-167
- G.C. Shah, Troubleshooting Reboiler Systems, Chemical and Engineering Progress, Juli 1979, 53-58
- Canadian Journal of Chemistry, Bd. 42, 1964, 818-824
- J. Chem. Thermodynamics, 1999, 31, 1077-1084
- Kopie der JPH8-151349 und englische Übersetzung davon
- Ullmann's Encyclopedia of Ind. Chem., 5. Aufl., Bd. 1, 1985, 161, 165-167
- Kirk-Othmer, Encyclopedia of Chem. Tech., 4. Aufl. Bd. 8, 1993, 311-312. 320-323, 349-352
- Kirk-Othmer, Encyclopedia of Chem. Tech., 4. Aufl., Bd. 1, 1991, 297-301
- John J. McKetta, Encyclopedia of Chem. Processing and Design, 46: Pumps, Bypass to Reboilers, 1994, 492-494, 519
- Exhibit A (HTRI simulation results) filed on 07.06.04
- Exhibit B (Output Summary) filed on 07.06.04

## Beschreibung

Vorliegende Erfindung betrifft ein Verfahren der kontinuierlichen destillativen Auftrennung von flüssigen Gemischen, die als Hauptbestandteil Acrylsäure enthalten, in einer Destillationsvorrichtung, die eine Destillierblase, einen Kondensator und eine Verbindung zwischen Destillierblase und Kondensator aufweist und der das aufzutrennende flüssige Gemisch kontinuierlich zugeführt wird.

Acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung und weist insbesondere im flüssigen Aggregatzustand eine hohe Polymerisationsneigung auf. Sichere Lagerung von im wesentlichen reiner flüssiger Acrylsäure ist selbst bei tiefen Temperaturen nur unter Zusatz von Polymerisationsinhibitor möglich.

Unter anderem ist Acrylsäure durch katalytische Gasphasenoxidation von Alkanen, Alkanolen, Alkenen oder Alkenalen erhältlich, die 3 C-Atome enthalten. Besonders vorteilhaft ist Acrylsäure z.B. durch katalytische Gasphasenoxidation von Propen oder Acrolein erhältlich.

Als Ausgangsverbindungen sind aber auch solche denkbar, aus welchen sich die eigentliche C₃-Ausgangsverbindung während der Gasphasenoxidation erst intermediär bildet.

Dabei werden diese Ausgangsgase, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende)Mischoxidkatalysatoren geleitet und oxidativ in die Acrylsäure umgewandelt (vgl. z.B. DE-A 44 05 059, EP-A 253 409, EP-A 92 097, DE-A 44 31 949).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine Acrylsäure , sondern ein Reaktionsgemisch erhalten, das im wesentlichen Acrylsäure, die inerten Verdünnungsgase und Nebenprodukte enthält, aus welchem die Acrylsäure abgetrennt werden muß.

Üblicherweise erfolgt die Abtrennung der Acrylsäure aus dem Reaktionsgemisch über extraktive und destillative Trennverfahren. D.h. in der Regel wird die gebildete Acrylsäure aus dem Reaktionsgasgemisch der Gasphasenoxidation zunächst in ein geeignetes Absorptionsmittel aufgenommen. Durch destillative Auftrennung des Absorbats wird dann üblicherweise eine Roh- Acrylsäure erhalten, aus der mittels Durchlaufen weiterer destillativer Trennstufen häufig eine Rein- Acrylsäure erzeugt wird (z.B. DE-A 44 36 243, DE-PS 21 36 396, DE-A 43 08 087, EP-A 297 445, EP-A 117 146, EP-B 102 642, GB-PS 1 346 737 und die DE-B 2 207 184).

Die Roh- Acrylsäure weist in der Regel eine Reinheit , ≥ 95 Gew.-%, häufig ≥ 97 Gew.-% auf, wobei sich die Verunreinigungen insbesondere aus niederen Aldehyden (z.B. Formaldehyd, Acetaldehyd, Acrolein, Propionaldehyd, Benzaldehyd, Furfural oder Crotonaldehyd), Wasser, niederen Alkancarbonsäuren (z.B. Essigsäure und Propionsäure) und Anhydriden von Alkancarbonsäure (z.B. Maleinsäureanhydrid) rekrutieren. Im Unterschied zur Roh- Acrylsäure liegt die Reinheit der Rein- Acrylsäure im Normalfall bei ≥ 98 Gew.-%, häufig bei ≥ 99 Gew.-%.

Das Problem der destillativen Auftrennung von flüssigen Gemischen, die als Hauptbestandteil (z.B. ≥ 95 Gew.-%) Acrylsäure enthalten, ist somit hinlänglich bekannt.

Üblicherweise erfolgt die destillative Auftrennung in kontinuierlicher Weise in einer Destillationsvorrichtung, die eine Destillierblase, einen Kondensator und eine Verbindung zwischen Destillierblase und Kondensator aufweist und der das aufzutrennende flüssige Gemisch kontinuierlich zugeführt wird.

Integraler Bestandteil der Destillierblase ist üblicherweise ein Verdampfer, der die zur Siedeverdampfung erforderliche Energie indirekt über beheizte Wärmeaustauschflächen an das destillativ aufzutrennende flüssige Gemisch überträgt.

Problematisch an der destillativen Auftrennung von flüssigen Gemischen, die als Hauptbestandteil Acrylsäure enthalten, ist, unabhängig davon, ob die Acrylsäure abtrennung über Kopf oder über Sumpf erfolgt, daß während der destillativen Auftrennung, auch bei Mitverwendung von Polymerisations-inhibitoren wie Luft, Hydrochinon, Hydrochinonmonomethylether, Paranitrosophenol, Paramethoxyphenol und/oder Phenothiazin, aufgrund der selbst bei reduziertem Druck erforderlichen hohen Siedeverdampfungstemperaturen in der Regel Polymerisatbildung erfolgt, die ursächlich dafür verantwortlich zeichnet, daß insbesondere die Wärmeaustauschfläche des Verdampfers relativ rasch mit Belag bedeckt. Dies hemmt u.a. den Wärmeübergang und mindert die Verdampferleistung, weshalb die kontinuierlich durchgeführte Destillation von Zeit zu Zeit zum Zwecke der Entfernung der Belagsbildung unterbrochen werden muß (insbesondere im Fall der Anwendung von Rohrbündelwärmeaustauschern, die infolge der Belagsbildung vollständig verstopfen können; in der Literatur (z.B. EP-A 648 520 und EP-A 616 998) werden deshalb bereits nicht destillative Verfahren zur Reinigung von Roh- Acrylsäure empfohlen).

Die Aufgabe der vorliegenden Erfindung bestand nun darin, ein neues Verfahren der kontinuierlichen destillativen Auftrennung von flüssigen Gemischen, die als Hauptbestandteil Acrylsäure enthalten, in einer Destillationsvorrichtung, die eine Destillierblase, einen Kondensator und eine Verbindung zwischen Destillierblase und Kondensator aufweist und der das aufzutrennende flüssige Gemisch kontinuierlich zugeführt wird, zur Verfügung zu stellen, das die Problematik der Belagsbildung auf der Verdampferoberfläche in reduziertem Ausmaß aufweist.

Demgemäß wurde ein Verfahren der kontinuierlichen destillativen Auftrennung von flüssigen Gemischen, die als Hauptbestandteil Acrylsäure enthalten, in einer Destillationsvorrichtung, die eine Destillierblase, einen Kondensator und eine verbindung zwischen Destillierblase und Kondensator aufweist und der das aufzutrennende flüssige Gemisch kontinuierlich zugeführt wird gefunden, wobei wenigstens ein Teil (in der Regel wenigstens 50 %, häufig wenigstens 75 % und besonders bevorzugt wenigstens 100 %) der zum Siedeverdampfen des flüssigen Gemisches erforderlichen Energie der Destillationsvorrichtung dadurch zugeführt wird, daß man dem bei einem Druck Pₓ befindlichen flüssigen Inhalt der Destillierblase kontinuierlich einen Anteil entnimmt, diesen bei einem oberhalb von Pₓ gelegenen Druck P_{y} auf eine Temperatur T_{y}, mit der Maßgabe erhitzt, daß T_{y}, oberhalb der zum Druck Pₓ gehörigen Siedetemperatur Tₓ und unterhalb der zum Druck P_{y} gehörigen Siedetemperatur T_{y} des flüssigen Inhalts der Destillierblase liegt, und anschließend den bezogen auf den Druck Pₓ so überhitzten entnommenen Anteil der Destillierblasenflüssigkeit unter Entspannung in die Destillationsvorrichtung zurückführt, wobei das Verfahren dadurch gekennzeichnet ist, dass T_{y}, 3 bis 12K oberhalb der Temperatur des flüssigen Inhalts der Destillierblase liegt.

Als weiterem Stand der Technik konnte von der Anwendung der Zwangsumlaufentspannungsverdampfung zur Minderung der Krustenbildung an Verdampferoberflächen im Fall der Eindampfung von Stoffgemischen, in welchen mit zunehmender Konzentrierung zunehmende Feststoffabscheidung erfolgt, ausgegangen werden (z.B. Verdampfertechnik von Reinhard Billet, Hochschultaschenbücher Verlag, Bibliographisches Institut-Mannheim (1965), S. 146ff, und Perry's Chemical Engineers Handbook, Sixth Edition, McGraw-Hill Book Company (1984), S. 11 bis 32, Heat Transfer).

Der wesentliche Unterschied zwischen dem erfindungsgemäßen Verfahren und der vorgenannten Anwendung der Zwangsumläufentspannungsverdampfung besteht jedoch darin, daß die Polymerisationsneigung von Acrylsäure mit steigender Temperatur wächst, wohingegen die Löslichkeit der meisten Stoffe entropiebedingt mit der Temperatur zunimmt. D.h., während das Erhitzen einer abgeschiedenen Feststoff enthaltenden Lösung den abgeschiedenen Feststoffanteil in der Lösung in der Regel reduziert; nimmt die Feststoffabscheidung in Acrylsäure als Hauptbestandteil enthaltenden flüssigen Gemischen mit der Temperatur zu. Das weiteren dürfte die Polymerisationsneigung von Acrylsäure im flüssigen Aggregatzustand mit zunehmendem Druck anwachsen. Angesichts dieses Sachverhalts muß die geschilderte Vorteilhaftigkeit des erfindungsgemäßen Verfahrens gegenüber den Verfahren des Standes der Technik überraschen.

Das erfindungsgemäße Verfahren entfaltet seine Vorteilhaftigkeit sowohl bei destillativ aufzutrennenden flüssigen Gemischen mit einem Acrylsäure anteil von ≥ 95, ≥ 96, ≥ 97, ≥ 98 als auch ≥ 99 Gew. -%. Es kann sowohl bei Normaldruck (1·10⁵ Pa (1 bar)) als auch bei reduziertem Druck durchgeführt werden. Vorzugsweise erfolgt die erfindungsgemäße destillative Auftrennung bei reduziertem Druck (besonders bevorzugt bei 0.01 0.150·10⁵ Pa (10 bis 150 mbar). Die erfindungsgemäße destillative Auftrennung kann sowohl als Einphasenstromauftrennung (einfache Destillation) als auch als Zweiphasenstromauftrennung (Rektifikation) ausgeführt werden. Dabei kann prinzipiell sowohl ein Zweiphasen-Gegenstrom (der aufsteigende Dampfstrom wird in einer Kolonne zum fallenden Rücklauf-Flüssigkeitsstrom im Gegenstrom geführt) als auch ein Zweiphasen-Gleichstrom (der aufsteigende Dampfstrom wird in einer Kolonne zum Rücklauf-Flüssigkeitsstrom im Gleichstrom geführt) verwirklicht werden..Im Fall einer erfindungsgemäßen Zweiphasenstromauftrennung wird die Verbindung zwischen Destillierblase und Kondensator in der Regel durch eine Einbauten aufweisende Rektifikationsvorrichtung gebildet. Als solche kommen alle an sich bekannten Kolonnentypen wie Bodenkolonnen, Füllkörperkolonnen oder Rotationskolonnen in Betracht.

Im Fall der erfindungsgemäßen Einphasenstromauftrennung ist die Verbindung zwischen Destillierblase und Kondensator in der Regel eine bis auf Prallvorrichtungen (verhindern das Mitreißen von Flüssigkeitströpfchen) im wesentlichen einbautenfreie Kolonne.

Die kontinuierliche Zufuhr des erfindungsgemäß destillativ aufzutrennenden, als Hauptbestandteil Acrylsäure enthaltenden. flüssigen Gemisches kann sowohl unmittelbar in die Destillierblase selbst als auch in die Verbindung zwischen Destillierblase und Kondensator erfolgen.

Insbesondere eignet sich das erfindungssgemäße Verfahren zur destillativen Reinigung von Roh- Acrylsäure wie sie auf dem an sich bekannten, eingangs beschriebenen, Weg der katalytischen Gasphasenoxidation anfällt Dabei können die die Polymerisationsneigung der Acrylsäure signifikant erhöhenden niederen aldehydischen Verunreinigungen in an sich bekannter Weise unter Zusatz von Hydrazin und/oder dessen Derivaten (z.B. Aminoguanidinhydrogencarbonat) reduziert werden. Ferner können in bekannter Weise Alkylbenzolsulfonsäureverbindungen wie Dodecylbenzolsulfonsäure zugesetzt werden, die die Neigung zur Belagsbildung mindern. Als Polymerisationsinhibitoren kommen die eingangs bereits genannten in Betracht, unter denen Phenothiazin bevorzugt wird. Sie werden in an sich bekannten Mengen (einige ppm (typisch: ca. 200 ppm) bezogen auf Acrylsäure) eingesetzt.

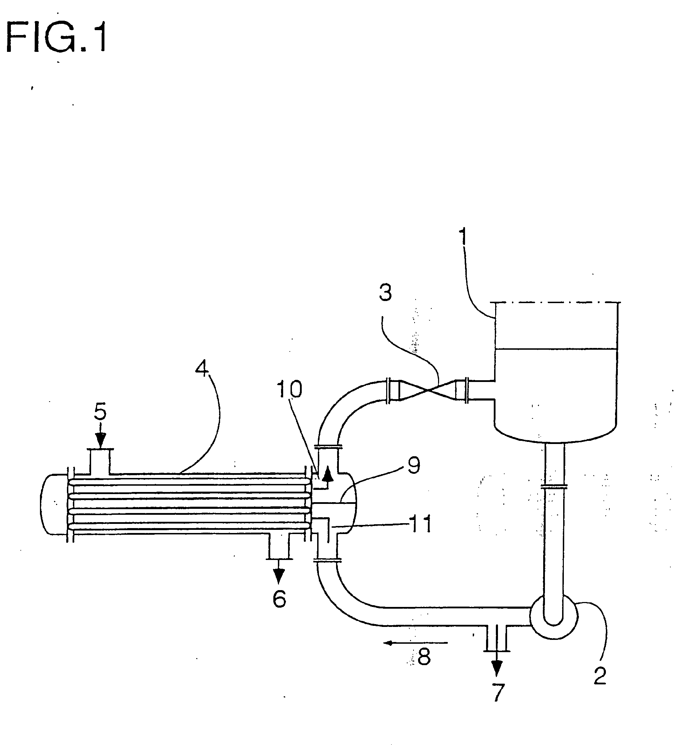

In besonders einfacher Weise läßt sich das erfindungsgemäße Verfahren in Anwendung eines außerhalb der Destillierblase angebrachten und von der Destillierblase durch eine Drosselvorrichtung getrennten Zwangsumlaufröhrenverdampfers realisieren, wie es in Figur 1 dargestellt ist (1=Destillierblase, 2=Umlaufpumpe, 3=Drosselvorrichtung, 4=Röhrenverdampfer, 5=Heizdampf, 6=Heizdampfkondensat, 7=Sumpfproduktentnahme, 8=Umlaufrichtung, 9=Trennvorrichtung, 10=Ausstrom, 11=Zustrom).

Dem bei einem Druck Pₓ befindlichen flüssigen Inhalt der Destillierblase wird kontinuierlich ein Teil entnommen und mittels einer Umlaufpumpe in die Zuströmrohre eines Röhrenverdampfers (Rohrbündelwärmeaustauscher) gepumpt. Um die innenliegenden Rohre des Röhrenverdampfers strömt ein Wärmeträger, z.B. Heizdampf (in der Regel unter Druck stehender Wasserdampf), dessen Temperatur oberhalb der Temperatur des Flüssiginhalts der Destillierblase liegt. Auf dem Weg durch die Zu- und Ausströmrohre des Röhrenverdampfers wird die entnommene Destillierblasenflüssigkeit durch indirekten Wärmeaustausch auf eine Temperatur T_{y}, erhitzt, die oberhalb der Temperatur des Flüssiginhalts der Destillierblase liegt. Eine Drosselvorrichtung trennt Röhrenverdampfer und Destillierblase druckseitig und ermöglicht durch geeignete Wahl der Umlaufpumpenleistung die Einstellung eines oberhalb von Pₓ gelegenen Drosselvordrucks P_{y}, der oberhalb des zur Temperatur T_{y'} gehörigen Siededrucks P_{y'} der entnommenen Destillierblasenflüssigkeit liegt.

Erfindungsgemäß wesentlich ist, daß gemäß vorstehenden Maßnahmen ein Sieden (Blasenbildung) des umgepumpten Destillierblasenflüssigkeitsteils in den Rohren des Röhrenverdampfers unterdrückt wird. Der umgepumpte Anteil der Destillierblasenflüssigkeit wird in den Rohren des Röhrenverdampfers bezüglich des über dem flüssigen Inhalt der Destillierblasen herrschenden Druck Pₓ vielmehr überhitzt und der Siedeprozeß so auf die Durchtrittsseite der Drosselvorrichtung verlagert (d.h. der Inhalt der Röhren des Röhrenverdampfers liegt einphasig vor, der Röhrenverdampfer fungiert lediglich als Überhitzer).

Der Durchtritt der überhitzten Flüssigkeit durch die Drosselvorrichtung in die Destillationsvorrichtung kann dabei unmittelbar in den flüssigen Inhalt der Destillierblase hinein erfolgen. Unter diesen Bedingungen entspricht die Temperatur des flüssigen Inhalts der Destillierblase regelmäßig der zum über der Destillierblasenflüssigkeit herrschenden Druck Pₓ gehörigen Siedetemperatur Tₓ.

Prinzipiell kann der Durchtritt der überhitzten Flüssigkeit durch die Drosselvorrichtung in die Destillationsvorrichtung aber auch oberhalb des Flüssigkeitsspiegels des flüssigen Destillierblaseninhaltes in die Verbindung zwischen Destillierblase und Kondensator hinein erfolgen. Unter diesen Bedingungen liegt die Temperatur des flüssigen Inhalts der Destillierblase regelmäßig unterhalb der zum über der Destillierblasenflüssigkeit herrschenden Druck Pₓ gehörigen Siedetemperatur Tₓ. Wesentlich ist, daß die Siedeverdampfungswirkung des außerhalb der Destilliervorrichtung angebrachten Röhrenverdampfers erst in der Destilliervorrichtung, d.h. außerhalb des Verdampfers, eintritt.

Die Drosselung kann z.B. mechanisch (Blenden, Ventile) und/oder hydrostatisch (durch eine entsprechend hohe Flüssigkeitssäule in der Destillierblase über der Durchtrittsstelle der überhitzten Flüssigkeit) erfolgen.

Erfindungsgemäß einzusetzende Röhrenverdampfer umfassen häufig 100 bis 300 Rohre aus V4A-Stahl. Die Dicke der Röhrenwände beträgt oft 1 bis 3 mm und der Außendurchmesser liegt regelmäßig bei 25 bis 35 mm. Ihre Länge kann bis zu einigen Metern betragen (typisch: 3 m). Als Wärmeträger umströmt die Rohre in der Regel unter Überdruck (bis zu einigen bar) stehender heißer Wasserdampf. Es können jedoch auch andere übliche Wärmeträger, wie wärmeträgeröle und/oder heiße Gase, verwendet werden. Die Leistung der Umlaufpumpe liegt typisch bei 100 000 bis 300 000 1/h. Dies bedingt Strömgungsgeschwindigkeiten in den Rohren des Röhrenverdampfers von 1 bis 7 m/sec.

Insgesamt wird die Wärmeübertragung im Röhrenverdampfer erfindungsgemäß so gestaltet, daß T_{y}, 3 bis 12 K oberhalb der Temperatur des flüssigen Inhalts der Destillierblase liegt. Die Differenz zwischen P_{y} und Pₓ wird in der Regel nicht mehr als 8·10⁵ Pa (8 bar), oft nicht mehr als 5·10⁵ Pa (5 bar) und häufig nicht mehr als 3·10⁵ Pa (3 bar) betragen. Vielfach beträgt sie 0.2·10⁵ bis 1·10⁵ Pa (0.2 bis 1 bar).

Die der Destillationsvorrichtung kontinuierlich zugeführte Menge an Acrylsäure als Hauptbestandteil enthaltendem flüssigen Gemisch liegt in typischer Weise bei 2000 bis 4000 l/h.

Von dem sich im Lauf der Zeit ansammelnden schwersiedenden Destillationsrückstand kann z.B. über die Entnahmestelle 7 kontinuierlich ein Teil entnommen werden.

Beispiel und Vergleichsbeispiele
a) Destillative Einphasenstromauftrennung einer durch gasphasenkatalytische Oxidation erhaltenen, mit Phenothiazin versetzten und mit Aminoguanidinhydrogencarbonat behandelten sowie mit Dodecylbenzolsulfonsäure versetzten Acrylsäure, die

| | |
|---|---|
| 99,25 Gew.-% | Acrylsäure |
| 0,2 Gew.-% | Dodecylbenzolsulfonsäure (Belagsbildungsminderer) |
| 500 ppm | Essigsäure |
| 200 ppm | Propionsäure |
| 10 ppm | niedermolekulare Aldehyde |
| 300 ppm | Wasser |
| 200 ppm | Phenothiazin (Prozeß-Polymerisationsinhibitor) und |
| 2000 ppm | Diacrylsäure |

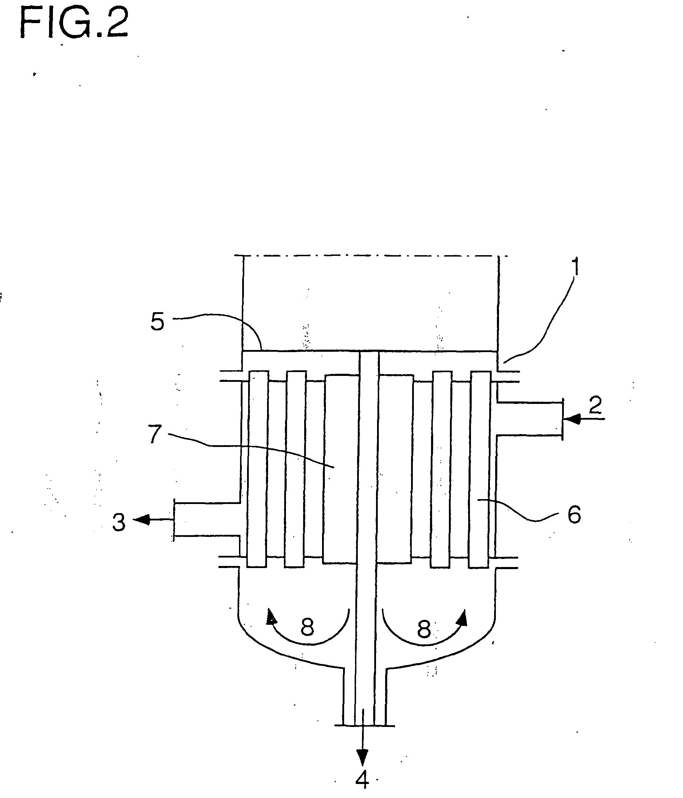

enthielt, in einer aus Destillierblase, Kondensationsvorrichtung und Verbindung zwischen Destillierblase und Kondensationsvorrichtung bestehenden Destillationsvorrichtung, wobei in die Destillierblase ein Robert-Verdampfer wie in Figur 2 integriert war (1 = Destillierblase, 2 = Heizdampf, 3 = Heizdampfkondensat, 4 =Sumpfproduktaustrag, 5 = Flüssigkeitsstand, 6 = Verdampferrohr, 7 = zentrales Fallrohr, 8 = Umlaufrichtung).
Die genauen Daten der Destillationsvorrichtung und der Betriebsweise lauteten:
- Einbautenfreie, lediglich Prallvorrichtungen aufweisende Destillationskolonne aus Edelstahl mit Innendurchmesser = 1000 mm und Länge = 4000 mm;
- Robert-Verdampfer mit 351 Verdampferrohren aus Edelstahl (Außendurchmesser der Verdampferrohre = 30 mm, Wanddicke = 2 mm, Länge = 1200 mm);
- Zulauf der aufzutrennenden Acrylsäure unmittelbar in die Destillierblase mit einer Zulauftemperatur von 25°C: 3000 1/h;
- Druck in der Kolonne : 0.07·10⁵ Pa (70 mbar);
- Siedetemperatur in den Verdampferrohren: 75°C;
- 4 bar Heizdampf einer Temperatur von 151°C;
- Dampfverbrauch: ca. 800 kg/h;
- Sumpfproduktentnahme: 300 l/h.

Im Kondensator fielen ca. 2700 1/h eines Kondensats an, das
99,7 Gew.-% Acrylsäure
< 1 ppm Phenothiazin
< 10 ppm niedermolekulare Aldehyde und
< 1000 ppm Diacrylsäure
   enthielt, und dem unmittelbar Hydrochinonmonomethylether als Lagerstabilisator zugesetzt wurde.

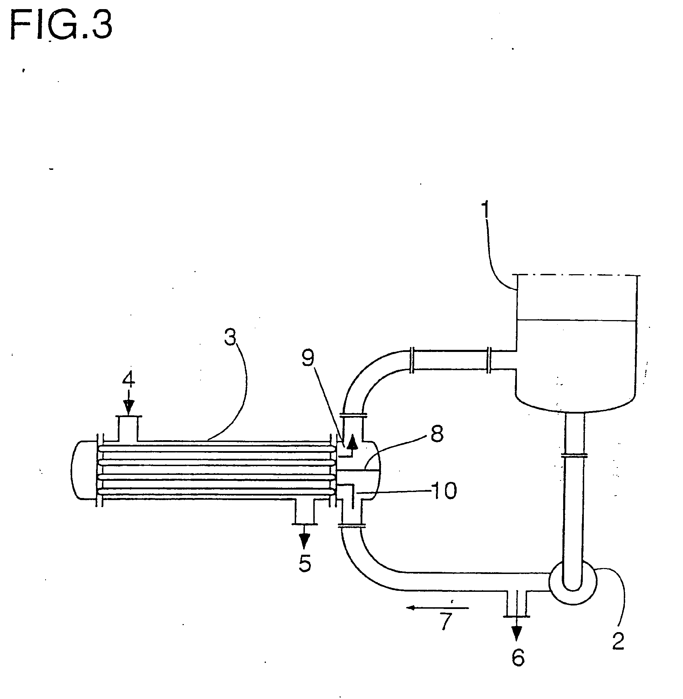

In den engen Verdampferrohren wird die aufzutrennende Acrylsäure von den entstehenden Siededampfblasen aufwärts gefördert und im zentralen Fallrohr wieder nach unten geführt (natürlicher Selbstumlauf).
Nach 7 Tagen kontinuierlichem Betrieb mußte die Destillationsvorrichtung wegen Verstopfung der Verdampferrohre abgeschaltet werden.
b) Destillative Einphasenstromauftrennung derselben Acrylsäure wie in a) in einer aus Destillierblase, Kondensationsvorrichtung und Verbindung zwischen Destillierblase und Kondensationsvorrichtung bestehenden Destillationsvorrichtung, wobei der Rohrbündelverdampfer aus der Destillierblase wie in Figur 3 ausgelagert war (1 = Destillierblase, 2. = Umlaufpumpe, 3 = Röhrenverdampfer, 4 = Heizdampf, 5 = Heizdampfkondensat, 6 = Sumpfproduktentnahme, 7 = Umlaufrichtung, 8 = Trennvorrichtung, 9 = Ausstrom, 10 = Zustrom). Die Förderung der aufzutrennenden Acrylsäure erfolgte mittels einer Umlaufpumpe.
Die genauen Daten der Destillationsvorrichtung und der Betriebsweise lauteten:
- Einbautenfreie, lediglich Prallvorrichtungen aufweisende Destillationskolonne aus Edelstahl mit Innendurchmesser = 1000 mm und Länge = 4000 mm;
- Ausgelagerter Röhrenverdampfer mit 219 Verdampferrohren aus Edelstahl (Außendurchmesser der Verdampferrohre = 30 mm, Wanddicke = 2 mm, Länge = 3000 mm);
- Förderleistung der Umwälzpumpe: 250 000 l/h;
- Strömungsgeschwindigkeit in den Verdampferrohren: 1,4 m/sec;
- Zulauf der aufzutrennenden Acrylsäure unmittelbar in die Destillierblase mit einer Zulauftemperatur von 25°C: 3000 l/h;
- Druck in der Kolonne: 0.07·10⁵ Pa (70 mbar);
- Siedetemperatur in den Verdampferrohren: 75,5°C (bedingt durch den zusätzlichen hydrostatischen Druck der Blasenflüssigkeit);
- 4 bar Heizdampf einer Temperatur von 151°C;
- Dampfverbrauch: ca. 800 kg/h;
- Sumpfproduktentnahme: 300 l/h;
- Kondensatanfall wie in a).

Die in den Verdampferrohren siedende Flüssigkeit wurde unmittelbar in die Destillierblasenflüssigkeit geführt. Nach 20 Tagen kontinuierlichem Betrieb mußte die Destillationsvorrichtung wegen Verstopfung der Verdampferrohre abgeschaltet werden.
c) Destillative Einphasenstromauftrennung derselben Acrylsäure wie in a) in einer Destillationsvorrichtung wie in b) mit dem Unterschied zu b), daß der Röhrenverdampfer von der Destillierblase druckseitig durch eine Drosselvorrichtung getrennt war. Der dadurch aufgebaute Druck im Röhrenverdampfer betrug vor der Drossel 3·10⁵ Pa (3 bar) (Pumpendruck) und verhinderte in selbigem Siedeblasenbildung. Die Differenz zwischen der Siedetemperatur in der Destillierblase (75°C) und der Temperatur in den Rohren des Röhrenverdampfers betrug 8 K. Der Eintritt der überhitzten Flüssigkeit erfolgte unmittelbar in die Destillierblasenflüssigkeit.

Nach 4 Monaten kontinuierlichem Betrieb wiesen die Rohre des Röhrenverdampfers noch keinen Belag auf.

## Patentansprüche

1. Verfahren der kontinuierlichen destillativen Auftrennung von flüssigen Gemischen, die als Hauptbestandteil Acrylsäure enthalten, in einer Destillationsvorrichtung, die eine Destillierblase, einen Kondensator und eine Verbindung zwischen Destillierblase und Kondensator aufweist und dar das aufzutrennende flüssige Gemisch kontinuierlich zugeführt wird, wobei wenigstens ein Teil der zum Siedeverdampfen des flüssigen Gemisches erforderlichen Energie der Destillationsvorrichtung dadurch zugeführt wird, dass man dem bei einem Druck Pₓ befindlichen flüssigen Inhalt der Destillierblase kontinuierlich einen Anteil entnimmt, diesen bei einem oberhalb von Pₓ gelegenen Druck P_{y} auf eine Temperatur T_{y} mit der Maßgabe erhitzt, dass T_{y} oberhalb der zum Druck Pₓ gehörigen Siedetemperatur Tₓ und unterhalb der zum Druck P_{y} gehörigen Siedetemperatur T_{y} des flüssigen inhalts der Destillierblase liegt und anschließend den bezogen auf den Druck Pₓ so überhitzten entnommenen Anteil der Destillierblassenflüsslgkelt unter Entspannung In die Destillationsvorrichtung zurückführt, **dadurch gekennzeichnet dass** T_{y}, 3 bis 12 K oberhalb der Temperatur des flüssigen Inhalts der Destillierblase liegt.

## Claims

1. A process for the continuous distillative separation of liquid mixtures which comprise acrylic acid as the main component, in a distillation apparatus which comprises a still, a condenser and a connection between still and condenser and to which the liquid mixture to be separated is continuously fed, wherein at least some of the energy required for evaporation of the liquid mixture is supplied to the distillation apparatus by a procedure in which a fraction of the liquid content present at a pressure Pₓ in the still is continuously removed, said fraction is heated to a temperature T_{y'} at a pressure P_{y} above Pₓ, with the proviso that T_{y'} is above the boiling point Tₓ, associated with the pressure Pₓ, and below the boiling point T_{y}, associated with the pressure P_{y}, of the liquid content of the still, and that fraction of the still liquid which has been removed and superheated in this manner based on the pressure Pₓ is then recycled to the distillation apparatus while letting down the pressure, wherein T_{y}, is from 3 to 12 K above the temperature of the liquid content of the still.

## Revendications

1. Procédé de séparation par distillation en mode continu de mélanges liquides qui contiennent comme constituant principal de l'acide acrylique, dans un appareil de distillation comportant un alambic, un condensateur et une liaison entre l'alambic et le condensateur et auquel est amené en continu le mélange liquide à séparer, au moins une partie de l'énergie nécessaire à l'évaporation par ébullition du mélange liquide étant amenée à l'appareil de distillation en ce que l'on prélève en continu une fraction au contenu liquide de l'alambic, et qui se trouve à une pression Pₓ, on chauffe celle-ci, à une pression P_{y} située au-dessus de Pₓ, à une température T_{y}, étant entendu que T_{y}, se situe au-dessus de la température d'ébullition Tₓ liée à la pression Pₓ et en dessous de la température d'ébullition T_{y} liée à la pression P_{y}, du contenu liquide de l'alambic, et on amène ensuite la fraction prélevée ainsi surchauffée, se rapportant à la pression Pₓ, du liquide de l'alambic, sous dilatation, à l'appareil de distillation, **caractérisé en ce que** T_{y'} est située de 3 à 12 K au-dessus de la température du contenu liquide de l'alambic.
